# FASCICULE DE BREVET EUROPEEN

(11) **EP 0 695 360 B1**
(45) Date de publication et mention de la délivrance du brevet: **05.07.2006**
(21) Numéro de dépôt: 94913649.3
(22) Date de dépôt: 15.04.1994
(51) Int. Cl.: C12N 15/86, A61K 48/00, C12N 15/12, C12N 15/11

(54) **ADENOVIRUS RECOMBINANTS DEFECTIFS POUR LA THERAPIE GENIQUE DES TUMEURS**
DEFEKTIVE REKOMBINANTE ADENOVIREN ZUR TUMOR-GENTHERAPIE
DEFECTIVE RECOMBINANT ADENOVIRUSES FOR GENE THERAPY OF TUMOURS

(30) Priorité: 22.04.1993 FR 9304745
(43) Date de publication de la demande: 07.02.1996
(60) Demande divisionnaire: 06001083.2
(73) Titulaire: Aventis Pharma S.A., 92160 Antony (FR)
(72) Inventeur: PERRICAUDET, Michel, F-28320 Ecrosnes (FR); HADDADA, Hedi, F-94140 Alfortville (FR); MAY, Evelyne, F-75011 Paris (FR)
(74) Mandataire: Le Coupanec, Pascale A.M.P.
(86) Numéro de dépôt international: PCT/FR1994/000421
(87) Numéro de publication internationale: WO 1994/024297

(56) Documents cités:
- WO-A-93/03769
- WO-A-93/19191
- WO-A-94/10323
- JOURNAL OF CELLULAR BIOCHEMISTRY vol. SUPPL, no. 17E , 29 Mars 1993 page 204 WILLS, K.N. & MENZEL, P. 'Adenovirus vectors for gene therapy of cancer' & Keystone Symposium on genetically targeted research and therapeutics. Antisense and gene therapy, Keystone, USA ;12 au 18 Avril 1993
- JOURNAL OF CELLULAR BIOCHEMISTRY vol. SUP, no. 18A , 4 Janvier 1994 page 237 GREGORY, R.J. ET AL. 'Tumor suppressor gene therapy of cancer: adenoviral mediated gene transfer of the p53 cDNA into human tumor'
- AMERICAN REVIEW OF RESPIRATORY DISEASES vol. 145, no. 4 P2 , Avril 1992 page A425 BRODY,S.L. ET AL. 'In vivo gene transfer to malignant mesothelioma using an adenovirus vector' & 1992 International Conference of the American Lung Association and the American Thoracic Society Miami Beach, USA; 17-20 Mai 1992
- SPITZ ET AL.: ANTICANCER RESEARCH, vol. 16, 1996, pages 3415-3422,
- LIU ET AL.: EXPERIMENTAL HEMATOLOGY, vol. 28, 2000, pages 1354-1362,

## Description

La présente invention concerne des vecteurs recombinants d'origine virale et leur utilisation pour le traitement des cancers. Plus particulièrement, elle concerne des adénovirus recombinants comportant une séquence d'ADN hétérologue dont l'expression dans une cellule se divisant anormalement permet d'inhiber au moins partiellement la division de ladite cellule. L'invention concerne également la préparation de ces vecteurs et les compositions pharmaceutiques les contenant.

La croissance cellulaire est régulée de manière extremement subtile par deux types de signaux. Certains favorisent la multiplication des cellules, tandis que d'autres, au contraire, les font entrer dans un état quiescent ou les font se différencier, selon les besoins de l'organisme. Les cancers sont tous caractérisés par un dérèglement des mécanismes contrôlant la division cellulaire, conduisant à une prolifération anormale. Le plus souvent, le développement d'un cancer implique donc l'activation de gènes favorisant la multiplication des cellules (gènes désignés proto-oncogènes, qui sont activés en oncogènes) et la disparition ou l'inactivation de gènes inhibant la prolifération cellulaire. La présente invention offre la possibilité de traiter les cancers par la thérapie génique, par l'administration, à des cellules tumorales, d'un ou plusieurs de ces gènes dont l'expression permet d'inhiber au moins partiellement la prolifération cellulaire.

La thérapie génique consiste à corriger une déficience ou une anormalité (mutation, expression aberrante, etc) par introduction d'une information génétique dans la cellule ou l'organe affecté. Cette information génétique peut être introduite soit in vitro dans une cellule extraite de l'organe, la cellule modifiée étant alors réintroduite dans l'organisme, soit directement in vivo dans le tissu approprié. Dans ce second cas, différentes techniques existent, parmi lesquelles des techniques diverses de transfection impliquant des complexes d'ADN et de DEAE-dextran (Pagano et al., J.Virol. 1 (1967) 891), d'ADN et de protéines nucléaires (Kaneda et al., Science 243 (1989) 375), d'ADN et de lipides (Felgner et al., PNAS 84 (1987) 7413), l'emploi de liposomes (Fraley et al., J.Biol.Chem. 255 (1980) 10431), etc. Plus récemment, l'emploi de virus comme vecteurs pour le transfert de gènes est apparu comme une alternative prometteuse à ces techniques physiques de transfection. A cet égard, différents virus ont été testés pour leur capacité à infecter certaines populations cellulaires. En particulier, les rétrovirus (RSV, HMS, MMS, etc), le virus HSV, les virus adéno-associés, et les adénovirus.

La possibilité d'utiliser la thérapie génique pour traiter les cancers a déjà été évoquée dans la demande WO91/15580. Cette demande décrit la construction de rétrovirus contenant un gène codant pour un ribozyme, dont l'expression en culture cellulaire peut permettre de détruire un ARNm d'un oncogène.

La présente invention résulte de la mise en évidence que les adénovirus constituent des vecteurs particulièrement efficaces pour le transfert et l'expression de gènes thérapeutiques dans les tumeurs. En particulier, les adénovirus présentent l'avantage de ne pas s'intégrer au génome des cellules qu'ils infectent, de s'y maintenir de manière très stable ce qui permet d'obtenir un effet thérapeutique durable, et d'avoir un spectre d'hôte très large, ce qui permet une application au traitement de cancers affectant tout type de cellules. Par ailleurs, l'invention repose également sur la mise en évidence que les virus de type adénovirus sont capables de transférer et d'exprimer des gènes capables d'inhiber au moins partiellement la division cellulaire directement au niveau de tumeurs.

Un premier objet de l'invention réside donc dans un adénovirus recombinant défectif contenant une séquence d'ADN hétérologue dont l'expression permet d'inhiber au moins partiellement la division cellulaire.

L'invention a également pour objet l'utilisation d'un tel adénovirus recombinant défectif pour la préparation d'une composition pharmaceutique destinée au traitement ou à la prévention des cancers.

Au sens de la présente invention, le terme "adénovirus défectif" désigne un adénovirus incapable de se répliquer de façon autonome dans la cellule cible. Généralement, le génome des adénovirus défectifs utilisés dans le cadre de la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par le gène inséré. Préférentiellement, le virus défectif conserve néanmoins les séquences de son génome qui sont nécessaires à l'encapsidation des particules virales.

Il existe différents sérotypes d'adénovirus, dont la structure et les propriétés varient quelque peu. Néanmoins, ces virus ne sont pas pathogènes pour l'homme, et notamment les sujets non immuno-déprimés. Parmi ces sérotypes, on préfère utiliser dans le cadre de la présente invention les adénovirus de type 2 ou 5 (Ad 2 ou Ad 5). Dans le cas des adénovirus Ad 5, les séquences nécessaires à la réplication sont les régions E1A et E1B.

Au sens de la présente invention, la séquence d'ADN hétérologue dont l'expression permet d'inhiber au moins partiellement la division cellulaire comprend de préférence au moins un gène choisi parmi les gènes suppresseur de tumeur (ou anti-oncogène) ou tout dérivé actif desdits gènes; les gène antisens, dont l'expression dans la cellule cible permet d'inhiber l'expression de gènes favorisant la division cellulaire; ou les gènes dont le produit d'expression induit une apoptose de la cellule infectée.

Parmi les gènes suppresseurs de tumeur utilisables dans le cadre de la présente invention, on peut citer plus particulièrement les gènes suivants :
- Gène p53 :
   Le gène p53 code pour une protéine nucléaire de 53 kDa. La forme mutée par délétion et/ou mutation de ce gène est impliquée dans le développement de la plupart des cancers humains (Baker et coll., Science 244 (1989) 217). Ses formes mutées sont également capables de coopérer avec les oncogènes ras pour transformer des fibroblastes murins. Le gène sauvage codant pour la p53 native inhibe en revanche la formation des foyers de transformation dans des fibroblastes de rongeurs transfectés avec diverses combinaisons d'oncogènes. Des données récentes soulignent que la protéine p53 pourrait être elle-même un facteur de transcription et stimuler l'expression d'autres gènes suppresseurs de tumeur.
- Gène Rb
   Le gène Rb détermine la synthèse d'une phosphoprotéine nucléaire de 927 acides aminés environ (Friend et coll., Nature 323 (1986) 643) dont la fonction est de réprimer la division des cellules en les faisant entrer en phase de quiescence. Des formes inactivées du gène Rb ont été mises en cause dans différentes tumeurs, et notamment dans les rétinoblastomes ou dans les cancers mésenchymateux comme les ostéosarcomes. La réintroduction de ce gène dans les cellules tumorales où il était inactivé produit un retour à l'état normal et une perte de la tumorigénicité (Huang et coll., Science 242 (1988) 1563). Récemment, il a été démontré que la protéine Rb normale, mais pas ses formes mutées, réprime l'expression du proto-oncogène c-fos, gène indispensable à la prolifération cellulaire.
- Gène rap 1A
   Le gène rap 1A (également désigné k-rev1) code pour une protéine de 21 kDa associée à la face interne de la membrane cytoplasmique. Cette protéine est capable, à des niveaux élevés, de réverter des cellules transformées exprimant les oncogènes ras mutés (Kitayama et coll., Cell 56 (1989) 77).
- Gène DCC
   Le gène DCC code pour une protéine homologue avec les protéines d'adhésion cellulaire de la famille N-CAM. Ce gène est très fréquemment délété dans les carcinomes du colon (Fearon et coll., Science 247 (1990) 49).
- Gènes k-rev2 et k-rev3
   Le gène k-rev2 code pour une protéine sécrétée de 60 acides aminés, et le gène k-rev3 code pour une version tronquée d'une protéine de la matrice extracellulaire. Ces deux gènes sont capables de réverter des cellules NIH 3T3 transformées par l'oncogène Ki-ras.

D'autres gènes peuvent être utilisés dans le cadre de la présente invention pour leur effet anti-tumoral, et notamment d'autres gènes suppresseurs de tumeur décrits dans la littérature, ou tout autre gène dont le produit d'expression peut induire l'apoptose cellulaire.

Comme indiqué plus haut, la séquence d'ADN hétérologue peut comporter le gène suppresseur de tumeur natif ou un dérivé actif dudit gène. Un tel dérivé peut être obtenu par mutation, délétion, substitution et/ou addition d'une ou plusieurs paires de bases dans la séquence du gène, selon les techniques classiques de biologie moléculaire. L'activité du dérivé ainsi obtenu peut ensuite être confirmée in vitro sur des tests connus de l'homme du métier, tels que ceux décrits dans les exemples.

Au sens de l'invention, la séquence d'ADN hétérologue peut également comprendre un gène antisens, dont l'expression dans la cellule cible permet de contrôler l'expression de gènes ou la transcription d'ARNm cellulaires codant pour des protéines favorisant la prolifération cellulaire. De tels gènes peuvent par exemple être transcrits, dans la cellule cible, en ARN complémentaires d'ARNm cellulaires et bloquer ainsi leur traduction en protéine.

Parmi les gènes antisens utilisables dans le cadre de l'invention, on peut citer plus particulièrement toute séquence antisens permettant de diminuer les niveaux de production des oncogènes ras, myc, fos, c-erb B, etc.

Généralement, la séquence d'ADN hétérologue comprend également des séquences promotrices permettant l'expression du ou des gènes capables d'inhiber au moins partiellement la division cellulaire dans la cellule cible. Il peut s'agir de séquences promotrices qui sont naturellement responsables de l'expression dudit gène lorsque ces séquences sont susceptibles de fonctionner dans la cellule infectée. Il peut également s'agir de séquences promotrices d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus, y compris l'adénovirus utilisé. A cet égard, on peut citer par exemple les promoteurs des gènes E1A, MLP, CMV, RSV, etc. En outre, ces séquences promotrices peuvent être modifiées par addition de séquences d'activation, de régulation, etc. Par ailleurs, lorsque la séquence d'ADN hétérologue ne comporte pas de séquences d'expression, elle peut être insérée dans le génome du virus défectif en aval d'une telle séquence. Il est également possible d'utiliser des promoteurs inductibles.

Par ailleurs, dans un autre mode de mise en oeuvre de l'invention, la séquence d'ADN hétérologue comprend, en plus du gène suppresseur de tumeur ou du gène antisens, un gène codant pour un antigène spécifique de tumeur et/ou un gène codant pour une lymphokine. La combinaison de ces gènes permet en effet (i) de stopper la division cellulaire dans une tumeur et ainsi, de faire régresser ladite tumeur, et (ii), d'augmenter la réponse immunitaire de l'organisme contre ladite tumeur.

Les antigènes spécifiques de tumeur sont des motifs antigéniques qui apparaissent à la surface de cellules tumorales, mais qui n'existent pas à la surface des mêmes cellules non tumorales. De tels antigènes sont généralement utilisés pour le diagnostic de cancers. Plus récemment, ils ont été décrits pour la réalisation de vaccins anti-tumoraux (EP 259 212). Cependant, ils n'ont jamais été combinés à d'autres gènes thérapeutiques comme dans le cadre de la présente invention.

Parmi les gènes codant pour des lymphokines, on peut citer plus particulièrement les gènes codant pour les interleukines (IL-1 à IL-13), les interférons, les facteurs de nécrose des tumeurs, les facteurs de stimulation des colonies (G-CSF, M-CSF, GM-CSF, etc), le TGFβ, etc. Par ailleurs, le gène codant pour la lymphokine comprend généralement, en amont de la séquence codante, une séquence d'expression et une séquence signal dir geant le polypeptide synthétisé dans les voies de sécrétion de la cellule cible. Cette séquence signal peut être la séquence signal naturelle de la lymphokine, mais il peut également s'agir de toute autre séquence signal fonctionnelle, ou d'une séquence signal artificielle. De telles constructions permettent en particulier d'augmenter les taux de lymphokine de manière très localisée, et ainsi, en présence d'un antigène spécifique de tumeur, d'amplifier la réponse immunitaire contre un type particuliuer de tumeur, ce qui donne un effet particulièrement avantageux. De tels adénovirus recombinants sont particulièrement utilisables pour la préparation de vaccins anti-tumoraux.

Les adénovirus recombinants défectifs selon l'invention peuvent être préparés par toute technique connue de l'homme du métier (Levrero et al., Gene 101 (1991) 195, EP 185 573; Graham, EMBO J. 3 (1984) 2917). En particulier, ils peuvent être préparés par recombinaison homologue entre un adénovirus et un plasmide portant entre autre la séquence d'ADN hétérologue. La recombinaison homologue se produit après co-transfection desdits adénovirus et plasmide dans une lignée cellulaire appropriée. La lignée cellulaire utilisée doit de préférence (i) être transformable par lesdits éléments, et (ii), comporter les séquences capables de complémenter la partie du génome de l'adénovirus défectif, de préférence sous forme intégrée pour éviter les risques de recombinaison. A titre d'exemple de lignée, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12 %).

Ensuite, les adénovirus qui se sont multipliés sont récupérés et purifiés selon les techniques classiques de biologie moléculaire, comme illustré dans les exemples.

La présente invention concerne également une composition pharmaceutique comprenant un ou plusieurs adénovirus recombinants défectifs tels que décrits précédemment. De préférence, les compositions pharmaceutiques de l'invention contiennent un véhicule pharmaceutiquement acceptable pour une formulation directement injectable dans les tumeurs à traiter. Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. L'injection directe dans la tumeur à traiter est avantageuse car elle permet de concentrer l'effet thérapeutique au niveau des tissus affectés.

Les doses d'adénovirus recombinant défectif utilisées pour l'injection peuvent être adaptées en fonction de différents paramètres, et notamment en fonction du mode d'administration utilisé, de la pathologie concernée, du gène à exprimer, ou encore de la durée du traitement recherchée. D'une manière générale, les adénovirus recombinants selon l'invention sont formulés et administrés sous forme de doses comprises entre 10⁴ et 10¹⁴ pfu/ml, et de préférence 10⁶ à 10¹⁰ pfu/ml. Le terme pfu ("plaque forming unit") correspond au pouvoir infectieux d'une solution de virus, et est déterminé par infection d'une culture cellulaire appropriée, et mesure, généralement après 48 heures, du nombre de plages de cellules infectées. Les techniques de détermination du titre pfu d'une solution virale sont bien documentées dans la littérature.

La présente invention offre ainsi un moyen très efficace pour le traitement ou la prévention des cancers. En outre, ce traitement peut concerner aussi bien l'homme que tout animal tel que les ovins, les bovins, les animaux domestiques (chiens, chats, etc), les chevaux, les poissons, etc.

La présente invention sera plus complètement décrite à l'aide des exemples qui suivent, qui doivent être considérés comme illustratifs et non limitatifs.

### Légende des figures

Figure 1 : Représentation du vecteur mp53wtI-.CMV
Figure 2 : Représentation du vecteur mp53pIX.CMV

### Techniques générales de biologie moléculaire

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropancl, la transformation dans Escherichia coli, etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y.. 1982; Ausubel F.M. et al. (eds), "Current Protocols in Molecular Biology", John Wiley & Sons, New York, 1987].

Les plasmides de type pBR322, pUC et les phages de la série M13 sont d'origine commerciale (Bethesda Research Laboratories).

Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant.

La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### Exemples

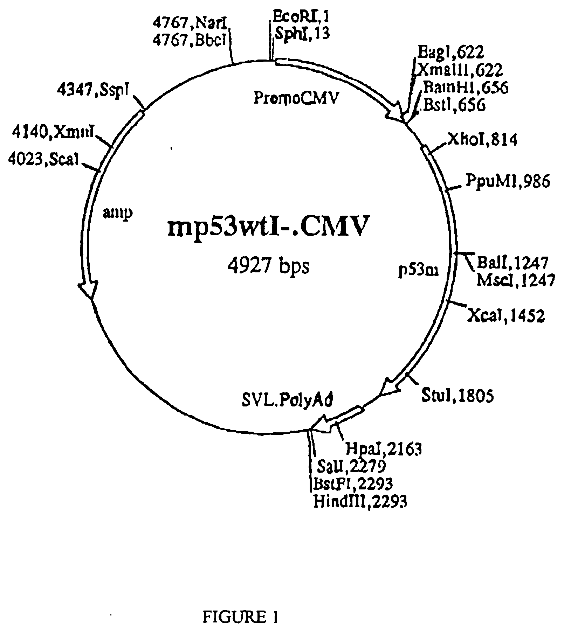

### EI. Construction du vecteur mp53wtI-CMV portant le gène p53 sous le contrôle du promoteur du cytomégalovirus (figure 1).

Le vecteur d'expreasion eucaryote mp53wtI-CMV a été construit à partir du plasmide pUC19, par insertion :
- d'une région promotrice d'origine virale qui correspond au promoteur précoce du cytomégalovirus (CMV). Cette région est entourée dans le vecteur de sites de restrictions uniques EcoRI-SphI à la jonction CMV / pUC et BamHI à la jonction CMV / p53. La présence de sites uniques flanquant la région promotrice permet de remplacer la région CMV par tout autre promoteur. Une deuxième série de vecteurs est ainsi obtenue dans laquelle le gène p53 est placé sous le contrôle d'un promoteur inductible : le promoteur de la metallothionine, inductible par les métaux lourds (cadnium et zinc).
- d'une séquence de 1173 pb correspondant à l'ADNc codant pour la protéine p53 de souris sous sa forme sauvage (Zakut-Houri et al., Nature 36 (1983) 594). Dans cette construction, le gène suppresseur est sous forme d'ADNc, c'est-à-dire dépourvue d'introns. Ceci permet notamment de réduire la taille du vecteur. Par ailleurs, il a été vérifié que les niveaux d'expression obtenus sont comparables en présence ou en l'absence d'introns.
- du signal de polyadénylation des gènes tardifs du virus SV40, qui correspond à un signal de polyadénylation très efficace. Deux sites de restriction uniques SalI et HindIII sont situés en aval du signal de polyadénylation. Ces sites ont permis l'insertion des régions pIX de l'adénovirus (Cf E3).

### E2. Activité in vitro du vecteur mp53wtI-CMV

La fonctionnalité du vecteur mp53wtI-CMV a été confirmée in vitro, par expression transitoire dans les cellules HeLa. Pour cela, le vecteur a été introduit dans les cellules par transfection et, 40 heures après, la protéine p53 a été dosée par immunofluorescence et immunoprécipitation. Les résultats obtenus montrent que plus de 50% des cellules transfectées induisent des taux importants de protéine p53.

### E3. Construction du vecteur mp53pIX.CMV

Les plasmides utilisés pour générer, par recombinaison homologue, les adénovirus recombinants exprimant le gène p53 ont été construits comme suit :

Le vecteur d'expression eucaryote mp53pIX.CMV a été construit par insertion de la séquence pIX issue du génome de l'adénovirus entre les sites SalI et EcoRI de mp13wtI-.CMV. La séquence pIX a été isolée à partir du plasmide recombinant pLTR-βgal pIX (Stratford-Perricaudet et al., J. Clin. Invest. 90 (1992) 626) par digestion au moyen des enzymes EcoRV et HindIII.

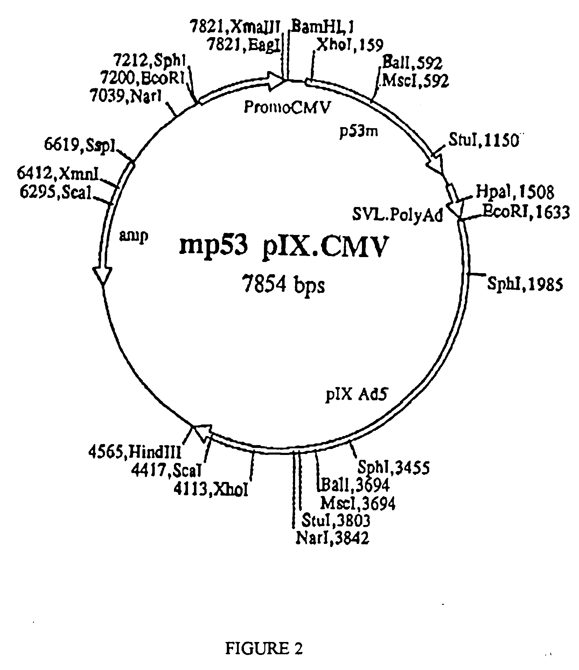

Le vecteur d'expression mp53pIX.CMV ainsi obtenu (figure 2) possède un site unique HindIII en aval de l'insert pIX ce qui permet une linéarisation de la construction (CfE4).

### E4. Construction d'un adénovirus recombinant défectif portant le gène p53 sous le contrôle du promoteur CMV.

Le vecteur mp53pIX.CMV est linéarisé et cotransfecté avec un vecteur adénoviral déficient, dans les cellules helper (lignée 293) apportant en *trans* les fonctions codées par les régions E1 (E1A et E11B) d'adénovirus.

On obtient l'adénovirus Ad.p53 par recombinaison homologue in vivo entre l'adénovirus mutant Ad-d1324 (Thimmappaya et al., Cell 31 (1982) 543) et le vecteur mp53pIX.CMV, selon le protocole suivant : après linéarisation par l'enzyme HindIII, le plasmide mp53pIX.CMV et l'adénovirus d1324 sont co-transfectés dans la lignée 293 en présence de phosphate de calcium, pour permettre la recombinaison homologue. Les adénovirus recombinants ainsi générés sont sélectionnés par purification sur plaque. Après isolement, l'ADN de l'adénovirus recombinant est amplifié dans la lignée cellulaire 293, ce qui conduit à un surnageant de culture contenant l'adénovirus défectif recombinant non purifié ayant un titre d'environ 10¹⁰ pfu/ml.

Les particules virales sont ensuite purifiées par centrifugation sur gradient de chlorure de césium selon les techniques connues (voir notamment Graham et al., Virology 52 (1973) 456). L'adénovirus Adp53 peut être conservé à -80°C dans 20 % de glycérol.

La capacité de l'adénovirus Ad-p53 à infecter des cellules en culture et à exprimer dans le milieu de culture une forme biologiquement active de la p53 sauvage a été démontrée par infection des cellules de la lignée 293 humaine. La présence de p53 dans le surnageant de culture a ensuite été mise en évidence au moyen d'un anticorps monoclonal spécifique de la p53.

Ces études permettent de montrer que l'adénovirus exprime bien une forme biologiquement active de la p53.

## Revendications

1. Adénoviras recombinant défectif contenant une séquence d'ADN hétérologue dont l'expression dans une cellule cible permet d'inhiber au moins partiellement la division cellulaire, ladite séquence comprenant
- au moins un gène codant pour la protéine p53 et,
- une séquence promotrice permettant l'expression, dans la cellule infectée, dudit gène, la dite séquence promotrice ayant une origine différente de celle dudit gène.

2. Adénavirus recombinant défectif selon la revendication 1 contenant une séquence codant pour la protéine p53 sauvage sous contrôle d'un promoteur hétérologue.

3. Adénovirus recombinant défectif selon la revendication 1 ou 2, **caractérisé en ce que** la séquence promotrice est une séquence promotrice issue du génome de la cellule infectée.

4. Adénovirus recombinant défectif selon la revendication 1 ou 2, **caractérisé en ce que** la séquence promotrice est une séquence synthétique.

5. Adénovirus recombinant défectif selon la revendication 1 ou 2, **caractérisé en ce que** la séquence promotrice est une séquence d'origine virale choisie parmi les séquences promotrices des gènes CMV ou RSV.

6. Adénovirus recombinant défectif selon la revendication 1 ou 2, contenant une séquence codant pour la protéine p53 sauvage sous contrôle du promoteur précoce du cytomégalovirus.

7. Adénovirus selon l'une des revendications 1 à 6, **caractérisé en ce qu'**il est dépourvu des régions de son génome qui sont nécessaires à sa réplication dans la cellule cible.

8. Adénovirus selon la revendication 7 **caractérisé en ce qu'**il s'agit d'un adénovirus de type Ad 5.

9. Adénovirus selon l'une des revendications 1 à 8, **caractérisé en ce que** la séquence d'ADN hétérologue comprend en outre un gène codant pour un antigène spécifique de tumeur et/ou un gène codant pour une lymphokine.

10. Utilisation d'un adénovirus selon l'une des revendications 1 à 9 pour la préparation d'une composition pharmaceutique destinée au traitement et/ou la prévention des cancers par introduction directe dans le tissu à traiter.

11. Utilisation d'un adénovirus selon l'une des revendications 1 à 9 pour la préparation d'une composition pharmaceutique destinée au traitement et/ou la prévention des cancers par administration directe dans la tumeur à traiter.

12. Utilisation d'un adénovirus selon la revendication 2 pour la préparation d'une composition pharmaceutique destinée au traitement et/ou à la prévention des cancers associés à la présence d'une forme mutée de p53.

13. Utilisation selon la revendication 10 ou 11 pour la préparation d'un vaccin anti-tumoral.

14. Composition pharmaceutique comprenant un ou plusieurs adénovirus recombinants défectifs selon l'une des revendications 1 à 9.

15. Composition pharmaceutique selon la revendication 14 **caractérisée en ce qu'**elle est sous forme injectable.

16. Composition pharmaceutique selon la revendication 14 **caractérisée en ce qu'**elle comprend entre 10⁴ et 10¹⁴ pfu/ml, et de préférence 10⁶ à 10¹⁰ pfu/ml adénovirus recombinants défectifs.

17. Utilisation d'un adénovirus recombinant défectif contenant une séquence d'ADN hétérologue codant pour la protéine p53 sauvage pour la préparation d'une composition pharmaceutique destinée à induire l'apoptose cellulaire.

## Claims

1. Defective recombinant adenovirus containing a heterologous DNA sequence whose expression in a target cell makes it possible to at least partially inhibit cell division, the said sequence comprising
- at least one gene encoding the p53 protein, and
- a promoter sequence allowing the expression, in the infected cell, of the said gene, the said promoter sequence having an origin different from that of the said gene.

2. Defective recombinant adenovirus according to Claim 1, containing a sequence encoding the wild-type p53 protein under the control of a heterologous promoter.

3. Defective recombinant adenovirus according to Claim 1 or 2, **characterized in that** the promoter sequence is a promoter sequence derived from the genome of the infected cell.

4. Defective recombinant adenovirus according to Claim 1 or 2, **characterized in that** the promoter sequence is a synthetic sequence.

5. Defective recombinant adenovirus according to Claim 1 or 2, **characterized in that** the promoter sequence is a sequence of viral origin chosen from the promoter sequences of the CMV or RSV genes.

6. Defective recombinant adenovirus according to Claim 1 or 2, containing a sequence encoding the wild-type p53 protein under the control of the cytomegalovirus early promoter.

7. Adenovirus according to one of Claims 1 to 6, **characterized in that** it lacks the regions of its genome which are necessary for its replication in the target cell.

8. Adenovirus according to Claim 7, **characterized in that** it is a type Ad 5 adenovirus.

9. Adenovirus according to one of Claims 1 to 8, **characterized in that** the heterologous DNA sequence comprises, in addition, a gene encoding a tumour-specific antigen and/or a gene encoding a lymphokine.

10. Use of an adenovirus according to one of Claims 1 to 9, for the preparation of a pharmaceutical composition intended for the treatment and/or the prevention of cancers by direct introduction into the tissue to be treated.

11. Use of an adenovirus according to one of Claims 1 to 9, for the preparation of a pharmaceutical composition intended for the treatment and/or prevention of cancers by direct administration into the tumour to be treated.

12. Use of an adenovirus according to Claim 2, for the preparation of a pharmaceutical composition intended for the treatment and/or the prevention of cancers associated with the presence of a mutated form of p53.

13. Use according to Claim 10 or 11, for the preparation of an antitumour vaccine.

14. Pharmaceutical composition comprising one or more defective recombinant adenoviruses according to one of Claims 1 to 9.

15. Pharmaceutical composition according to Claim 14, **characterized in that** it is in injectable form.

16. Pharmaceutical composition according to Claim 14, **characterized in that** it comprises between 10⁴ and 10¹⁴ pfu/ml, and preferably 10⁶ to 10¹⁰ pfu/ml of defective recombinant adenoviruses.

17. Use of a defective recombinant adenovirus containing a heterologous DNA sequence encoding the wild-type p53 protein, for the preparation of a pharmaceutical composition intended to induce cell apoptosis.

## Patentansprüche

1. Defektes rekombinantes Adenovirus, das eine heterologe DNA-Sequenz enthält, durch deren Expression in einer Zielzelle sich die Zellteilung mindestens teilweise hemmen lässt, wobei die Sequenz einschließt
- mindestens ein Gen, das das Protein p53 codiert und
- eine Promotorsequenz, die die Expression des Gens in der infizierten Zelle erlaubt, wobei die Promotorsequenz einen von demjenigen des Gens unterschiedlichen Ursprung aufweist.

2. Defektes rekombinantes Adenovirus nach Anspruch 1, das eine Sequenz enthält, die das Wildtyp-Protein p53 unter der Kontrolle eines heterologen Promotors codiert.

3. Defektes rekombinantes Adenovirus nach Anspruch 1 oder 2, **dadurch gekennzeichnet, dass** die Promotorsequenz eine Promotorsequenz ist, die dem Genom der infizierten Zelle entstammt.

4. Rekombinantes defektes Adenovirus nach Anspruch 1 oder 2, **dadurch gekennzeichnet, dass** die Promotorsequenz eine synthetische Sequenz ist.

5. Defektes rekombinantes Adenovirus nach Anspruch 1 oder 2, **dadurch gekennzeichnet, dass** die Promotorsequenz eine Sequenz viralen Ursprungs ist, ausgewählt aus den Promotorsequenzen der CMV- oder RSV-Gene.

6. Defektes rekombinantes Adenovirus nach Anspruch 1 oder 2, das eine Sequenz enthält, die das Wildtyp-Protein p53 unter der Kontrolle des frühen Promotors des Cytomegalovirus codiert.

7. Adenovirus nach einem der Ansprüche 1 bis 6, **dadurch gekennzeichnet dass** ihm Regionen seines Genoms fehlen, die für seine Replikation in der Zielzelle notwendig sind.

8. Adenovirus nach Anspruch 7, **dadurch gekennzeichnet, dass** es sich um ein Adenovirus vom Typ Ad5 handelt.

9. Adenovirus nach einem der Ansprüche 1 bis 8, **dadurch gekennzeichnet, dass** die heterologe DNA-Sequenz ferner ein Gen einschließt, das ein spezifisches Tumor-Antigen und/oder ein Gen, das ein Lymphokin codiert, einschließt.

10. Verwendung eines Adenovirus nach einem der Ansprüche 1 bis 9 zur Herstellung einer pharmazeutischen Zubereitung, die zur Behandlung und/oder Prävention von Krebs durch direktes Einbringen in das zu behandelnde Gewebe bestimmt ist.

11. Verwendung eines Adenovirus nach einem der Anspräche 1 bis 9 zur Herstellung einer pharmazeutischen Zubereitung, die zur Behandlung und/oder Prävention von Krebs durch direkte Verabreichung in den zu behandelnden Tumor bestimmt ist.

12. Verwendung eines Adenovirus nach Anspruch 2 zur Herstellung einer pharmazeutischen Zubereitung, die zur Behandlung und/oder Prävention von Krebs bestimmt ist, der mit der Gegenwart einer mutierten Form von p53 zusammenhängt.

13. Verwendung nach Anspruch 10 oder 11 zur Herstellung eines Antitumor-Impfstoffes.

14. Pharmazeutische Zubereitung, umfassend ein oder mehrere defekte rekombinante Adenoviren nach einem der Ansprüche 1 bis 9.

15. Pharmazeutische Zubereitung nach Anspruch 14, **dadurch gekennzeichnet, dass** sie in injizierbarer Form vorliegt.

16. Pharmazeutische Zubereitung nach Anspruch 14, **dadurch gekennzeichnet, dass** sie zwischen 10⁴ und 10¹⁴ pfu/ml und vorzugsweise 10⁶ bis 10¹⁰ pfu/ml defekte rekombinante Adenoviren einschließt.

17. Verwendung eines defekten rekombinanten Adenovirus, der eine heterologe DNA-Sequenz enthält, die das Wildtyp-Protein p53 codiert, zur Herstellung einer pharmazeutischen Zubereitung, die zum Auslösen der Zellapoptosis bestimmt ist.
